# FASCICULE DE BREVET EUROPEEN

(11) **EP 0 663 910 B1**
(45) Date de publication et mention de la délivrance du brevet: **12.02.1997**
(21) Numéro de dépôt: 93921987.9
(22) Date de dépôt: 04.10.1993
(51) Int. Cl.: C07D 305/14

(54) **PROCEDE D'OBTENTION DE LA DESACETYL-10 BACCATINE III**
VERFAHREN ZUR GEWINNUNG VON 10-DEACETYLBACCATIN-III
METHOD OF OBTAINING 10-DEACETYLBACCATINE III

(30) Priorité: 05.10.1992 FR 9211746
(43) Date de publication de la demande: 26.07.1995
(73) Titulaire: RHONE-POULENC RORER S.A., 92160 Antony (FR)
(72) Inventeur: GAULLIER, Jean-Claude, F-77420 Champs-sur-Marne (FR); MANDARD, Bernadette, F-41110 Mareuil sur Cher (FR); MARGRAFF, Rodolphe, F-91170 Viry-Châtillon (FR)
(74) Mandataire: Pilard, Jacques
(86) Numéro de dépôt international: FR9300972
(87) Numéro de publication internationale: WO9407882

(56) Documents cités:
- EP-A- 0 553 780
- JOURNAL OF NATURAL PRODUCTS, vol. 49, no. 4 , Juillet 1986 Columbus, Ohio, US, pages 665 - 669 C.H.O. HUANG, ET AL.: 'New taxanes from Taxus brevifolia, 2' cité dans la demande
- COMPTES RENDUS DE L'ACADEMIE DES SCIENCES. SERIE II: MECANIQUE, PHYSIQUE, CHIMIE, SCIENCES DE L'UNIVERS, SCIENCES DE LA TERRE, vol. 299, no. 15 , 21 Novembre 1984 Montreuil, FR, pages 1039 - 1043 V. SENILH, ET AL.: 'Hémisynthèse de nouveaux analogues du taxol. Etude de leur interaction avec la tubulin'

## Description

La présente invention concerne un procédé pour obtenir sélectivement des intermédiaires utiles pour la préparation par des procédés semi-synthétiques du taxol, du Taxotère ou de leurs analogues à partir des différentes parties de plantes contenant ces intermédiaires.

Plus particulièrement, l'invention concerne l'obtention de la désacétyl-10 baccatine III à partir de l'écorce, du tronc, des racines ou des feuilles de différentes espèces d'ifs.

Le taxol et le Taxotère ainsi que leurs analogues de formule générale : 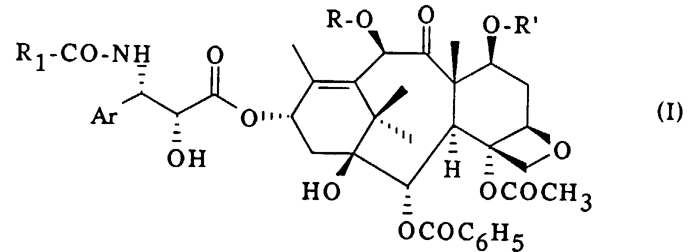 qui manifestent des propriétés anticancéreuses et antileucémiques remarquables, constituent des agents chimiothérapeutiques remarquables pour le traitement d'un certain nombre de cancers tels que par exemple les cancers du sein, de la prostate, du colon, de l'estomac, du rein ou des testicules et plus spécialement le cancer de l'ovaire.

Notamment, dans la formule générale (I), Ar peut représenter un radical phényle éventuellement substitué, R peut représenter un atome d'hydrogène ou un radical acétyle ou un radical carbamoyle N-substitué, R' représente un atome d'hydrogène ou un radical carbamoyle N-substitué et R₁ peut représenter un radical phényle ou un radical R₂-O- dans lequel R₂ représente un radical alcoyle, alcényle, alcynyle, cycloalcoyle, cycloalcényle, bicycloalcoyle, phényle ou hétérocyclyle.

Le taxol correspond au produit de formule générale (I) dans laquelle Ar et R₁ représentent un radical phényle et R représente un radical acétyle et R' représente un atome d'hydrogène et le Taxotère corespond au produit de formule générale (I) dans laquelle Ar représente un radical phényle, R et R' représentent un atome d'hydrogène et R₁ représente un radical t.butoxy.

Le taxol, qui existe à l'état naturel dans différentes espèces d'ifs dans lesquelles il se trouve en faibles quantités, est difficile à isoler sans procéder à la destruction complète de la plante. Par exemple, le taxol peut être isolé selon la méthode de C.H.O. HUANG et coll. J. Natl. Prod., 49, 665 (1986) qui consiste à traiter l'écorce broyée de Taxus brevifolia par le méthanol, à concentrer l'extrait, extraire le concentrat par le dichlorométhane, à concentrer à nouveau, à disperser le résidu dans un mélange hexane-acétone (1-1 en volumes), à purifier la partie soluble par chromatographie sur une colonne de Florisil pour obtenir le taxol brut qui est purifié par recristallisations successives dans des mélanges méthanol-eau et hexane-acétone puis par chromatographie et nouvelle cristallisation. Les quantités de taxol ainsi extraites peuvent représenter de 0,005 à 0,017 % de la partie de la plante mise en oeuvre.

Le Taxotère qui n'existe pas à l'état naturel peut être préparé par hémi-synthèse à partir de la désacétyl-10 baccatine III de formule : 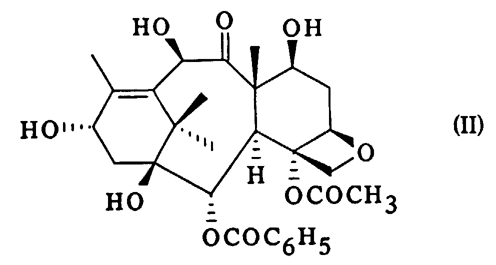 selon les procédés qui sont décrits, par exemple, dans les brevets américains US 4,814,470 ou US 4,924 ,012 ou dans la demande internationale PCT WO 92/09589.

Le taxol peut aussi être préparé par des procédés qui font intervenir la mise en oeuvre de la désacétyl-10 baccatine III soit par passage par l'intermédiaire du Taxotère dans les conditions décrites dans le brevet américain US 4,857,653 soit par estérification de la baccatine III dans les conditions décrites dans les brevets européens EP 400,971 ou EP 428,376 soit par estérification de la désacétyl-10 baccatine III et acétylation dans les conditions décrites dans les brevet américain US 4,924,011.

Les différentes variétés d'ifs (Taxus baccata, Taxus brevifolia, Taxus canadensis, Taxus cupidata,Taxus floridana, Taxus media et Taxus wallichiana) contiennent des dérivés du taxane dont les principaux sont essentiellement le taxol et la désacétyl-10 baccatine III, les autres dérivés étant plus particulièrement la céphalomannine, la désacétyl-10 céphalomannine ou la baccatine III éventuellement liés à des sucres.

Alors que le taxol se trouve principalement dans le tronc et l'écorce, la désacétyl-10 baccatine III est présente essentiellement dans les feuilles. Par ailleurs, la teneur en désacétyl-10 baccatine III dans les feuilles est généralement très supérieure à celle du taxol, que celui-ci soit présent dans l'écorce, le tronc ou dans les feuilles.

Il en résulte qu'il est particulièrement important de pouvoir disposer de désacétyl-10 baccatine III qui est essentielle à la préparation de quantités de taxol beaucoup plus importantes que par extraction directe à partir des ifs ainsi qu'à la préparation du Taxotère.

En extrayant la désacétyl-10 baccatine III à partir des feuilles d'ifs, on n'est pas conduit à une destruction totale de la plante dont les feuilles peuvent être utilisées à nouveau après chaque cycle de croissance.

Généralement, les méthodes connues d'extraction des dérivés du taxane contenus dans les différentes parties de l'if (écorce, tronc, racines, feuilles, ....) nécessitent la mise en oeuvre de techniques chromatographiques longues et coûteuses qui ne permettent pas une séparation totale et quantitative des dérivés du taxane présents initialement dans la plante.

Selon le procédé décrit dans le brevet américain US 4,814,470 qui met en oeuvre une macération des aiguilles dans l'éthanol, une extraction par un solvant organique tel que le chlorure de méthylène et des chromatographies successives, il est possible d'isoler environ 40% de la désacétyl-10 baccatine III contenue dans les feuilles.

Dans le brevet européen EP 0 553 780 est décrit un procédé de préparation du taxol et de la désacétyl-10 baccatine III à partir des racines de différentes espèces de Taxus après extraction méthanolique.

Les différents constituants dérivés du taxane présents dans les différentes parties de l'if peuvent aussi être séparés par des méthodes mettant en oeuvre la chromatographie en phase liquide "reverse" qui sont décrites, en particulier dans la demande international PCT WO 92/07842. Ces procédés consistent essentiellement à traiter les extraits bruts d'ifs par chromatographie en phase liquide "reverse" sur un adsorbant sur lequel se fixent les dérivés du taxane, à éluer les dérivés du taxane et à les isoler. Selon ce procédé, il est possible d'isoler environ 25 % de la désacétyl-10 baccatine III contenue dans les feuilles.

Il a maintenant été trouvé, et c'est ce qui fait l'objet de la présente invention, que la désacétyl-10 baccatine III peut être extraite sélectivement à partir des différentes parties de l'if, et plus particulièrement des feuilles, par un procédé simple ne mettant pas en oeuvre des techniques chromatographiques. Par exemple, il est possible d'extraire environ 75 % de la désacétyl-10 baccatine III présente dans les feuilles.

Plus particulièrement, le procédé selon l'invention consiste :
- ou bien
   1) à traiter les parties broyées de l'if (Taxus sp.) par un alcool aliphatique de façon à obtenir un extrait alcoolique contenant la désacétyl-10 baccatine III,
   2) à diluer à l'eau l'extrait alcoolique éventuellement concentré,
   3) à séparer par filtration, décantation ou centrifugation les insolubles présents dans la solution hydro-alcoolique obtenue,
   4) à éliminer la quasi totalité de l'alcool de la solution hydro-alcoolique,
   5) à extraire la désacétyl-10 baccatine III de la phase aqueuse ainsi obtenue par un solvant organique convenable,
   6) à éliminer le solvant de l'extrait organique ainsi obtenu contenant la désacétyl-10 baccatine III,
   7) à faire cristalliser sélectivement la désacétyl-10 baccatine III, à partir du résidu ainsi obtenu, dans un solvant organique,
   8) à isoler la désacétyl-10 baccatine III purifiée.
- ou bien
   1) à traiter les parties broyées de l'if (Taxus sp.) par un alcool aliphatique de façon à obtenir un extrait alcoolique contenant la désacétyl-10 baccatine III,
   2) à extraire l'extrait alcoolique par un solvant organique,
   3) à séparer la phase hydro-alcoolique obtenue par décantation,
   4) à diluer par l'eau la phase hydro-alcoolique,
   5) à séparer par filtration, décantation ou centrifugation les insolubles présents dans la solution hydro-alcoolique obtenue,
   6) à extraire la désacétyl-10 baccatine III de la phase hydro-alcoolique filtrée par un solvant organique,
   7) à éliminer totalement ou partiellement le solvant de l'extrait organique contenant la désacétyl-10 baccatine III,
   8) à faire cristalliser sélectivement la désacétyl-10 baccatine III, à partir de la solution ou du résidu obtenu, dans un solvant organique,
   9) à isoler la désacétyl-10 baccatine m purifiée

Le procédé selon l'invention peut être mis en oeuvre sur toute partie appropriée de l'if telle que l'écorce, le tronc, les racines ou les feuilles. L'if utilisé pour la mise en oeuvre du procédé selon l'invention appartient de préférence à la variété Taxus baccata, Taxus brevifolia, Taxus canadensis, Taxus cupidata, Taxus floridana, Taxus media ou Taxus wallichiana. Il est particulièrement avantageux d'utiliser les feuilles d'if (Taxus baccata, Taxus brevifolia) qui généralement sont plus riches en désacétyl-10 baccatine III. Pour une meilleure mise en oeuvre du procédé, il est préférable d'utiliser les différentes parties de l'if sous forme broyée et éventuellement séchée. Les fragments utilisés peuvent avoir des dimensions variant de 0,5 à quelques millimètres. Pour des questions de commodité, il peut être avantageux d'utiliser des fragments dont les dimensions moyennes sont inférieures à 1 mm. Les parties broyées et éventuellement séchées de l'if peuvent être obtenues par des opérations de broyage et éventuellement de séchage qui, éventuellement, précèdent ou suivent des opérations de congélation et de décongélation des parties fraîches de la plante ou s'intercalent dans des opérations de congélation et de décongélation des parties fraîches de la plante.

L'extrait alcoolique est obtenu en agitant un mélange, éventuellement chauffé, des parties broyées et éventuellement séchées de l'if avec un alcool généralement choisi parmi le méthanol, l'éthanol, le propanol, l'isopropanol et le t.butanol. Il est particulièrement avantageux d'utiliser le méthanol.

L'extrait alcoolique contenant la désacétyl-10 baccatine III peut être traité selon l'une des méthodes suivantes :
1) l'extrait alcoolique contenant la désacétyl-10 baccatine III est dilué par addition d'eau pour donner une solution hydro-alcoolique.

Pour la mise en oeuvre du procédé, il est avantageux de se placer dans des conditions particulières de dilution et de teneur en alcool dans la phase aqueuse, de façon à éviter les pertes en désacétyl-10 baccatine III et à éliminer la plus grande quantité possible de produits insolubles. Dans ces conditions, il peut être nécessaire de concentrer l'extrait alcoolique contenant la désacétyl-10 baccatine III préalablement à la dilution par l'eau.

Plus particulièrement, la dilution par addition d'eau dans l'extrait alcoolique éventuellement concentré par distillation, de préférence sous pression réduite, doit être faite de telle manière que le rapport entre le poids du mélange diluant et le poids de la matière sèche contenue dans l'extrait alcoolique soit compris entre 4 et 8, le mélange diluant eau-alcool contenant 10 à 30 % en poids d'alcool.

Les produits insolubles dans la solution hydro-alcoolique ainsi obtenue sont éliminés selon les techniques habituelles et de préférence par filtration, par décantation ou par centrifugation. Lorsque l'élimination est effectuée par filtration, il peut être avantageux d'opérer en présence d'un agent de filtration tel que la terre d'infusoire (célite) et d'un agent floculant.

De préférence, l'élimination de l'alcool de la solution hydro-alcoolique ainsi obtenue est effectuée par distillation, de préférence sous pression réduite, éventuellement en présence d'un agent antimousse, de façon à éviter ou à limiter la dégradation thermique des constituants du milieu.

Généralement, la désacétyl-10 baccatine III contenue dans solution aqueuse ainsi obtenue, dans laquelle la teneur en alcool est généralement inférieure à 1 %, est extraite, en une ou plusieurs fois, par un solvant organique choisi parmi les éthers tels que le méthyl t.butyléther, l'éthyl t.butyléther, le méthyl n.butyléther, le méthyl n.amyléther, l'éthyl t.amyléther, le t.butyl isopropyléther, l'éthyl isobutyléther, le t.butyl n.propyléther ou l'éthyl n.hexyléther et les esters aliphatiques tels que l'acétate d'éthyle, l'acétate de propyle, l'acétate d'isopropyle, l'acétate de n.butyle, l'acétate de t.butyle, le t.butylacétate de méthyle, le propionate de t.butyle ou l'acétate de t.amyle. D'un intérêt tout particulier sont le méthyl t.butyléther, l'éthyl t.butyléther, l'acétate d'éthyle ou l'acétate de n.butyle. Plus spécialement encore, il est avantageux d'utiliser l'acétate d'éthyle ou l'acétate de n.butyle,
2) l'extrait alcoolique, qui contient de l'eau provenant des feuilles d'if mises en oeuvre, est extrait par un solvant organique approprié choisi parmi les hydrocarbures aromatiques tels que le toluène ou le xylène.

L'extrait hydro-alcoolique est dilué par addition d'eau, de façon que l'extrait contienne de 20 à 40 % d'alcool, puis il est filtré ou décanté ou centrifugé pour séparer les insolubles présents. La solution hydro-alcoolique obtenue qui contient la désacétyl-10 baccatine III est extraite en une ou plusieurs fois, par un solvant organique choisi parmi les éthers tels que le méthyl t.butyléther, l'éthyl t.butyléther, le méthyl n.butyléther, le méthyl n.amyléther, l'éthyl t.amyléther, le t.butyl isopropyléther, l'éthyl isobutyléther, le t.butyl n.propyléther ou l'éthyl n.hexyléther et les esters aliphatiques tels que l'acétate d'éthyle, l'acétate de propyle, l'acétate d'isopropyle, l'acétate de n.butyle, l'acétate de t.butyle, le t.butylacétate de méthyle, le propionate de t.butyle ou l'acétate de t.amyle. D'un intérêt tout particulier sont le méthyl t.butyléther, l'éthyl t.butyléther, l'acétate d'éthyle ou l'acétate de n.butyle. Plus spécialement encore, il est avantageux d'utiliser l'acétate d'éthyle ou l'acétate de n.butyle,

Les extraits organiques, obtenus selon l'une ou l'autre voie, sont éventuellement lavés au moyen d'une solution aqueuse d'une base faible (solution aqueuse de carbonate de sodium par exemple) et/ou à l'eau. Après séchage éventuel, le solvant organique de l'extrait est éliminé, totalement ou partiellement, selon les méthodes habituelles et en particulier par distillation éventuellement sous pression réduite, pour donner une solution ou résidu généralement solide dont on isole la désacétyl-10 baccatine III.

La cristallisation sélective de la désacétyl-10 baccatine III est effectuée à partir d'une solution dans un solvant organique ou dans un mélange de solvants organiques. Comme solvants permettant la cristallisation sélective de la désacétyl-10 baccatine III peuvent être avantageusement utilisés les nitriles tels que l'acétonitrile ou le propionitrile, éventuellement en mélange avec un alcool aliphatique tel que le méthanol, l'éthanol, le propanol, l'isopropanol ou le n.butanol ou un ester aliphatique tel que l'acétate d'éthyle, l'acétate d'isopropyle, l'acétate de n.butyle ou l'acétate de t.butyle ou une cétone telle que l'acétone, la méthyl éthylcétone, la méthyl propylcétone, la méthyl n.buylcétone et la méthyl isobutylcétone. Il est particulièrement avantageux d'effectuer la cristallisation sélective dans l'acétonitrile éventuellement en présence d'éthanol et/ou d'acétone et/ou d'acétate d'éthyle et/ou d'acétate de n.butyle.

La désacétyl-10 baccatine III qui précipite peut être isolée par filtration, décantation ou centrifugation.

La désacétyl-10 baccatine III obtenue selon la procédé d'extraction de la présente invention peut être utilisée pour préparer le taxol ou le Taxotère ou leurs dérivés dans les conditions décrites plus particulièrement dans les brevets EP 0 253 738, EP 0 253 739, EP 0 336 840, EP 0 336 841, WO 92 09589, EP 0 400 971 et EP 0 428 376.

Les exemples suivants illustrent le procédé selon l'invention.

### EXEMPLE 1

Dans un percolateur, on place 200 litres de méthanol, 1,4 kg de laine de verre et 250 kg de feuilles d'if (Taxus baccata) broyées dont la taille moyenne des particules est voisine de 0,8 mm. On ajoute 400 litres de méthanol pur. La percolation est effectuée en alimentant le percolateur en solvant frais par le haut, au débit de 870 litres/heure, et en recueillant l'extrait méthanolique au bas du percolateur. La percolation dure 5 heures à 20°C. La solution méthanolique recueillie est évaporée sous pression réduite (70-80 kPa) à 40°C dans un évaporateur pour obtenir un concentrat dont la teneur en matière séche est comprise entre 40 et 80 % en poids.

On utilise un extrait méthanolique (préparé dans les conditions décrites ci-dessus) contenant 353 g d'extrait sec, 420 g de méthanol et 40 g d'eau, la quantité de désacétyl-10 baccatine III présente étant de 1130 mg.

Dans un réacteur de 2 litres, on introduit l'extrait méthanolique puis, en agitant, 1590 cm3 d'eau. Le mélange est agité pendant 1 heure puis on ajoute successivement 35 g de célite en agitant pendant 1 heure et 25 cm3 de floculant "ZETAG 87". La suspension finale obtenue est filtrée sur verre fritté N°4 de 130 mm de diamètre. Les produits insolubles et la célite sont lavés par 100 g d'un mélange eau-méthanol (8-2 en poids).

Le filtrat est placé dans un réacteur de 2 litres puis on ajoute 2 cm3 de SILICONE 426R. Le mélange est distillé sous pression réduite (7,3-11 kPa) à une température comprise entre 35 et 42°C, la température du bain extérieur étant comprise entre 45 et 50°C. On obtient un distillat (909 g) et un concentrat (1432 g) contenant 0,5 % de méthanol.

Le concentrat est extrait 1 fois par 700 cm3 d'acétate d'éthyle puis 2 fois par 350 cm3 d'acétate d'éthyle. Les phases organiques réunies sont lavées 2 fois par 700 cm3 d'une solution 0,1 M de carbonate de sodium puis par 1 fois 350 cm3 d'eau. Le pH est égal à 8. La phase organique (1049 g) est concentrée à sec sous pression réduite (1,33-21,3 kPa) à 40°C. On obtient ainsi 16,2 g d'extrait sec.

### EXEMPLE 2

Dans un ballon de 500 cm3, on introduit 45,5 g d'extrait sec contenant 9,3 % de désacétyl-10 baccatine III obtenu dans les conditions décrites dans l'exemple 1 puis on ajoute 18 cm3 d'acétate d'éthyle. Le mélange homogène est agité et chauffé à 50°C. On ajoute alors 136 cm3 d'acétonitrile en 15 minutes à 50°C. La suspension est agitée pendant environ 1 heure puis elle est refroidie à une température voisine de 20°C en environ 3 heures. Le précipité est séparé par filtration sur verre fritté N°3 (diamètre : 35 mm) puis il est lavé par 20 cm3 d'acétonitrile puis par 2 fois 20 cm3 de diisopropyléther. Le produit est séché sous pression réduite (1,33 kPa) à 40°C pendant 20 heures. On obtient ainsi 5,2 g d'un produit blanc contenant 75,8 % de désacétyl-10 baccatine III.

Le rendement de la cristallisation est de 93,6 %.

### EXEMPLE 3

Dans un ballon de 1 litre, on introduit 275,2 g d'une solution dans l'acétate d'éthyle de 39,6 g de matière sèche contenant 3,48 g de désacétyl-10 baccatine III. Cette solution est concentrée sous pression réduite (6,0 kPa). On recueille 164 g d'acétate d'éthyle. Au concentrat, on ajoute 36 cm3 d'éthanol puis on refroidit à 10°C. On ajoute alors lentement (1/2 heure environ) en agitant (30 tours/minute) 60 cm3 d'acétonitrile. La suspension est agitée pendant 1 heure à 10°C puis on ajoute rapidement 84 cm3 d'acétonitrile. On agite pendant 15 heures à 10°C. Le précipité est séparé par filtration sur verre fritté N° 3 (diamètre : 35 mm) puis il est lavé par 2 fois 20 cm3 d'acétonitrile puis par 2 fois 20 cm3 de diisopropyléther. Le produit est séché pendant 12 heures sous pression réduite (0,27 kPa) à 40°C. On obtient ainsi 2,85 g d'une poudre blanche cristallisée contenant 93,8 % de désacétyl-10 baccatine III.

Les eaux-mères de cristallisation et les lavages (247 g) sont concentrés sous pression réduite (2,7 kPa) à 40°C. On obtient ainsi 36 g de substance sèche qui est reprise par 18 cm3 d'éthanol. On ajoute alors lentement 72 cm3 d'acétonitrile. Le mélange est agité pendant 15 heures à un température voisine de 20°C. Le précipité est séparé par filtration et est lavé par 2 fois 5 cm3 d'acétonitrile et par 2 fois 5 cm3 de diisopropyléther. Après séchage, on obtient 0,662 g d'une poudre fine contenant 62,7 % de désacétyl-10 baccatine III.

### EXEMPLE 4

On utilise un extrait méthanolique préparé dans les conditions décrites dans l'exemple 1 constitué de 1840 g d'extrait sec, de 1117 g de méthanol et de 329 g d'eau et contenant 7,17 g de désacétyl-10 baccatine III.

Dans un réacteur de 10 litres, on introduit l'extrait méthanolique puis on l'extrait 3 fois par 1,5 litre de toluène sous forte agitation. La phase hydrométhanolique est séparée (3020 g) par décantation, diluée par addition d'eau (3800 g) puis filtrée. On sépare ainsi 42 g de résidu.

La solution hydro-méthanolique filtrée est extraite 3 fois par 1540 cm3 d'acétate de n.butyle. Les phases organiques réunies sont concentrées sous pression réduite (1,33 kPa) à 40°C de façon à obtenir une solution contenant 30 % d'extrait sec puis filtrées. Le résidu obtenu (4 g) est lavé par 50 cm3 d'acétate de n.butyle. Les filtrats organiques sont réunis et concentrés à nouveau sous pression réduite (1,33 kPa) à 40°C de façon à obtenir une solution contenant 65 % d'extrait sec (soit 95 g).

Le concentrat ainsi obtenu est introduit dans un ballon de 500 cm3. On ajoute 45 cm3 d'éthanol. Le mélange homogène est agité et chauffé à 40°C. On ajoute alors en 1 heure, à 40°C, 270 cm3 d'acétonitrile. La suspension est refroidie à -10°C, tout en agitant, en 2 heures puis maintenue à -10°C pendant une nuit. Le précipité est séparé parr filtration sur verre fritté, lavé 2 fois par 35 cm3 d'acétonitrile puis par 2 fois 35 cm3 de diisopropyléther puis séché pendant une nuit sous pression réduite (1,33 kPa) à 40°C.

On obtient ainsi 5,92 g d'un produit blanc contenant 84,2 % de désacétyl-10 baccatine III.

Le rendement, par rapport à la désacétyl-10 baccatine III contenue dans l'extrait méthanolique initial , est de 69,5 %.

## Revendications

1. Procédé d'obtention de la désacétyl-10 baccatine III à partir des différentes parties de l'if (Taxus sp.) caractérisé en ce que :
1) on traite les parties broyées de l'if par un alcool aliphatique de façon à obtenir un extrait alcoolique contenant la désacétyl-10 baccatine III
2) on dilue à l'eau l'extrait alcoolique éventuellement concentré,
3) on sépare par filtration, décantation, centrifugation les insolubles présents dans la solution hydro-alcoolique obtenue,
4) on élimine la quasi totalité de l'alcool de la solution hydro-alcoolique obtenue
5) on extrait la désacétyl-10 baccatine III contenue dans la phase aqueuse ainsi obtenue par un solvant organique convenable,
6) on élimine le solvant de l'extrait organique ainsi obtenu,
7) on cristallise sélectivement la désacétyl-10 baccatine III à partir du résidu ainsi obtenu, puis
8) on isole la désacétyl-10 baccatine III purifiée.

2. Procédé d'obtention de la désacétyl-10 baccatine III caractérisé en ce que :
1) on traite les parties broyées de l'if par un alcool aliphatique de façon à obtenir un extrait alcoolique contenant la désacétyl-10 baccatine III,
2) on extrait l'extrait alcoolique par un solvant organique,
3) on sépare la phase hydro-alcoolique obtenue par décantation,
4) dilue à l'eau la phase hydro-alcoolique,
5) on sépare par filtration décantation ou centrifugation les insolubles présents dans la solution hydro-alcoolique obtenue,
6) on extrait la désacétyl-10 baccatine III de la phase hydro-organique filtrée par un solvant organique,
7) on élimine totalement ou partiellement le solvant de l'extrait organique contenant la désacétyl-10 baccatine III,
8) on fait cristalliser sélectivement la désacétyl-10 baccatine III à partir de la solution ou du résidu obtenu dans un solvant organique, et
9) on isole la désacétyl-10 baccatine III.

3. Procédé selon l'une des revendications 1 ou 2 caractérisé en ce que l'on obtient l'extrait alcoolique en agitant les parties broyées et éventuellement séchées de l'if dans un alcool aliphatique choisi parmi le méthanol, l'éthanol, le propanol, l'isopropanol et le t.butanol.

4. Procédé selon la revendication 3 catactérisé en ce que l'alcool aliphatique est le méthanol.

5. Procédé selon l'une des revendications 1 ou 2 caractérisé en ce que les parties broyées et éventuellement séchées de l'if sont obtenues par des opérations de broyage et éventuellement de séchage qui, éventuellement précèdent ou suivent des opérations de congélation et de décongélation des parties fraîches de la plante ou s'intercalent dans des opérations de congélation et de décongélation des parties fraîches de la plante.

6. Procédé selon l'une des revendications 1, 3, 4 ou 5 caractérisé en ce que l'on dilue à l'eau l'extrait méthanolique éventuellement concentré de façon que le rapport entre le poids du mélange diluant et le poids de la matière sèche contenu dans l'extrait alcoolique soit compris entre 4 et 8 et que le mélange diluant eau-alcool contienne 10 à 30 % d'alcool pour obtenir une solution hydro-alcoolique.

7. Procédé selon l'une des revendications 1, 3, 4 ou 5 caractérisé en ce que les insolubles sont séparés de la solution hydro-alcoolique par filtration, décantation ou centrifugation.

8. Procédé selon l'une des revendications 1, 3, 4 ou 5 caractérisé en ce que l'on élimine l'alcool de la solution hydro-alcoolique par distillation éventuellement sous pression réduite.

9. Procédé selon l'une des revendications 1, 3, 4 ou 5 caractérisé en ce que l'on extrait la désacétyl-10 baccatine III de la phase aqueuse, obtenue après élimination de l'alcool, par un solvant organique.

10. Procédé selon la revendication 9 caractérisé en ce que le solvant organique est choisi parmi les les éthers et les esters aliphatiques.

11. Procédé selon la revendication 10 caractérisé en ce que les ethers sont choisis parmi le méthyl t.butyléther, l'éthyl t.butyléther, le méthyl n.butyléther, le méthyl n.amyléther, l'éthyl t.amyléther, le t.butyl isopropyléther, l'éthyl isobutyléther, le t.butyl n.propyléther et l'éthyl n.hexyléther.

12. Procédé selon la revendication 11 caractérisé en ce que les esters aliphatiques sont choisis parmi l'acétate d'éthyle, l'acétate de propyle, l'acétate d'isopropyle, l'acétate de n.butyle, l'acétate de t.butyle, le t.butylacétate de méthyle, le propionate de t.butyle et l'acétate de t.amyle.

13. Procédé selon la revendication 9 caractérisé en ce que le solvant organique est l'acétate d'éthyle ou l'acétate de n.butyle.

14. Procédé selon l'une des revendications 2, 3, 4 ou 5 caractérisé en ce que l'on extrait la phase alcoolique par un solvant organique choisi parmi les hydrocarbures aromatiques.

15. Procédé selon la revendication 11 caractérisé en ce que l'hydrocarbure aromatique est choisi parmi le toluène et le xylène.

16. Procédé selon l'une des revendications 2, 3, 4 ou 5 caractérisé en ce que l'on dilue la phase hydro-alcoolique séparée par décantation par addition d'eau de façon que la teneur en alcool dans la solution soit comprise entre 20 et 40 % en poids.

17. Procédé selon l'une des revendications 2, 3, 4 ou 5 caractérisé en ce que les insolubles sont séparés de la solution hydro-alcoolique diluée par filtration, décantation ou centrifugation.

18. Procédé selon l'une des revendications 2, 3, 4 ou 5 caractérisé en ce que l'on extrait la désacétyl-10 baccatine III de la phase hydro-alcoolique par un solvant organique.

19. Procédé selon la revendication 18 caractérisé en ce que le solvant est choisi parmi les éthers et les esters aliphatiques.

20. Procédé selon la revendication 19 caractérisé en ce que les ethers sont choisis parmi le méthyl t.butyléther, l'éthyl t.butyléther, le méthyl n.butyléther, le méthyl n.amyléther, l'éthyl t.amyléther, le t.butyl isopropyléther, l'éthyl isobutyléther, le t.butyl n.propyléther et l'éthyl n.hexyléther.

21. Procédé selon la revendication 20 caractérisé en ce que les esters aliphatiques sont choisis parmi l'acétate d'éthyle, l'acétate de propyle, l'acétate d'isopropyle, l'acétate de n.butyle, l'acétate de t.butyle, le t.butylacétate de méthyle, le propionate de t.butyle et l'acétate de t.amyle.

22. Procédé selon la revendication 20 caractérisé en ce que le solvant organique est l'acétate d'éthyle ou l'acétate de n.butyle.

23. Procédé selon l'une des revendications 2, 3, 4 ou 5 caractérisé en ce que l'extrait organique contenant la désacétyl-10 baccatine III est concentré totalement ou partiellement par distillation éventuellement sous pression réduite.

24. Procédé selon l'une des revendications 1, 2, 3, 4 ou 5 caractérisé en ce que l'on cristallise sélectivement la désacétyl-10 baccatine III dans un solvant organique choisi parmi les nitriles aliphatiques éventuellement en mélange avec un alcool aliphatique ou un ester aliphatique ou une cétone aliphatique.

25. Procédé selon la revendication 24 caractérisé en ce que les nitriles aliphatiques sont choisis parmi l'acétonitrile et le propionitrile.

26. Procédé selon la revendication 24 caractérisé en ce que l'alcool aliphatique est choisi parmi le méthanol, l'éthanol, le propanol, l'isopropanol et le n.butanol.

27. Procédé selon la revendication 24 caractérisé en ce que l'ester aliphatique est choisi parmi l'acétate d'éthyle, l'acétate d'isopropyle, l'acétate de n.butyle et l'acétate de t.butyle.

28. Procédé selon la revendication 24 caractérisé en ce que la cétone est choisie parmi l'acétone, la méthyl éthylcétone, la méthyl propylcétone, la méthyl n.buylcétone et la méthyl isobutylcétone.

29. Procédé selon la revendication 24 caractérisé en ce que la cristallisation sélective est effectuée dans l'acétonitrile éventuellement associé à l'éthanol et/ou l'acétate d'éthyle ou de n.butyle et/ou l'acétone

30. Procédé selon l'une des revendications 1, 2, 3, 4 ou 5 caractérisé en ce que la désacétyl-10 baccatine III purifiée est isolée par filtration, décantation ou centrifugation.

31. Procédé selon l'une des revendications 1 à 30 caractérisé en ce que l'on extrait la désacétyl-10 baccatine III à partir des feuilles de l'if.

32. Procédé selon la revendication 31 caractérisé en ce que l'if appartient à la variété Taxus baccata, Taxus brevifloria, Taxus canadensis, Taxus cupidata, Taxus floridana, Taxus media ou Taxus wallichiana.

## Patentansprüche

1. Verfahren zur Gewinnung von 10-Desacetyl-Baccatin III aus verschiedenen Teilen der Eibe (Taxus sp.), dadurch gekennzeichnet, daß man
1) die zerkleinerten Teile der Eibe mit einem aliphatischen Alkohol in der Weise behandelt, daß man einen das 10-Desacetyl-Baccatin III enthaltenden alkoholischen Extrakt gewinnt,
2) den gegebenenfalls konzentrierten alkoholischen Extrakt mit Wasser verdünnt,
3) die in der erhaltenen wäßrig-alkoholischen Lösung vorliegenden unlöslichen Anteile mittels Filtration, Dekantieren oder Zentrifugieren abtrennt,
4) praktisch den gesamten Alkohol aus der erhaltenen wäßrig-alkoholischen Lösung entfernt,
5) das in der auf diese Weise erhaltenen wäßrigen Phase enthaltene 10-Desacetyl-Baccatin III durch ein geeignetes organisches Lösungsmittel extrahiert,
6) das Lösungsmittel aus dem auf diese Weise erhaltenen organischen Extrakt entfernt,
7) das 10-Desacetyl-Baccatin III ausgehend von dem auf diese Weise erhaltenen Rückstand selektiv kristallisiert, und anschließend
8) das gereinigte 10-Desacetyl-Baccatin III isoliert.

2. Verfahren zur Gewinnung von 10-Desacetyl-Baccatin III, dadurch gekennzeichnet, daß man
1) die zerkleinerten Teile der Eibe mit einem aliphatischen Alkohol in der Weise behandelt, daß man einen das 10-Desacetyl-Baccatin III enthaltenden alkoholischen Extrakt gewinnt,
2) den alkoholischen Extrakt mit einem organischen Lösungsmittel extrahiert,
3) die erhaltene wäßrig-alkoholische Phase mittels Dekantieren abtrennt,
4) die wäßrig-alkoholische Phase mit Wasser verdünnt,
5) die in der erhaltenen wäßrig-alkoholischen Lösung vorliegenden unlöslichen Anteile mittels Filtration, Dekantieren oder Zentrifugieren abtrennt,
6) das 10-Desacetyl-Baccatin III aus der filtrierten wäßrig-organischen Phase durch ein organisches Lösungsmittel extrahiert,
7) das Lösungsmittel vollständig oder teilweise aus dem das 10-Desacetyl-Baccatin III enthaltenden organischen Extrakt entfernt,
8) das 10-Desacetyl-Baccatin III ausgehend von der erhaltenen Lösung oder dem erhaltenen Rückstand in einem organischen Lösungsmittel selektiv kristallisiert, und
9) das 10-Desacetyl-Baccatin III isoliert.

3. Verfahren nach einem der Ansprüche 1 oder 2, dadurch gekennzeichnet, daß man den alkoholischen Extrakt unter Rühren der zerkleinerten und gegebenenfalls getrockneten Teile der Eibe in einem aliphatischen Alkohol erhält, ausgewählt unter Methanol, Ethanol, Propanol, Isopropanol und tert.-Butanol.

4. Verfahren nach Anspruch 3, dadurch gekennzeichnet, daß der aliphatische Alkohol Methanol ist.

5. Verfahren nach einem der Ansprüche 1 oder 2, dadurch gekennzeichnet, daß die zerkleinerten und gegebenenfalls getrockneten Teile der Eibe durch die Arbeitsgänge der Zerkleinerung und gegebenenfalls der Trocknung erhalten werden, die gegebenenfalls vor oder nach den Arbeitsgängen des Einfrierens und des Auftauens der frischen Pflanzenteile oder unter Einfügung in diese Arbeitsgänge des Einfrierens oder des Auftauens der frischen Pflanzenteile durchgeführt werden.

6. Verfahren nach einem der Ansprüche 1, 3, 4 oder 5, dadurch gekennzeichnet, daß man den gegebenenfalls konzentrierten methanolischen Extrakt in der Weise mit Wasser verdünnt, daß das Verhältnis zwischen dem Gewicht der Verdünnungsmischung und dem Gewicht der in dem alkoholischen Extrakt enthaltenen Trockensubstanz zwischen 4 und 8 liegt und daß die Verdünnungsmischung Wasser/Alkohol 10 bis 30 % Alkohol enthält, um eine wäßrig-alkoholische Lösung zu erhalten.

7. Verfahren nach einem der Ansprüche 1, 3, 4 oder 5, dadurch gekennzeichnet, daß die unlöslichen Anteile von der wäßrig-alkoholischen Lösung durch Filtration, Dekantieren oder Zentrifugieren abgetrennt werden.

8. Verfahren nach einem der Ansprüche 1, 3, 4 oder 5, dadurch gekennzeichnet, daß man den Alkohol aus der wäßrig-alkoholischen Lösung durch Destillation, gegebenenfalls unter reduziertem Druck, entfernt.

9. Verfahren nach einem der Ansprüche 1, 3, 4 oder 5, dadurch gekennzeichnet, daß man das 10-Desacetyl-Baccatin III aus der nach der Entfernung des Alkohols erhaltenen wäßrigen Phase mit Hilfe eines organischen Lösungsmittels extrahiert.

10. Verfahren nach Anspruch 9, dadurch gekennzeichnet, daß das organische Lösungsmittel unter den Ethern und den aliphatischen Estern ausgewählt wird.

11. Verfahren nach Anspruch 10, dadurch gekennzeichnet, daß die Ether unter Methyl-tert.-butylether, Ethyl-tert.-butylether, Methyl-n-butylether, Methyl-n-amylether, Ethyl-tert.-amylether, tert.-Butyl-isopropylether, Ethyl-isobutylether, tert.-Butyl-n-propylether und Ethyl-n-hexylether ausgewählt werden.

12. Verfahren nach Anspruch 11, dadurch gekennzeichnet, daß die aliphatischen Ester unter Ethylacetat, Propylacetat, Isopropylacetat, n-Butylacetat, tert.-Butylacetat, Methyl-tert.-butylacetat, tert.-Butylpropionat und tert.-Amylacetat ausgewählt werden.

13. Verfahren nach Anspruch 9, dadurch gekennzeichnet, daß das organische Lösungsmittel Ethylacetat oder n-Butylacetat ist.

14. Verfahren nach einem der Ansprüche 2, 3, 4 oder 5, dadurch gekennzeichnet, daß man die alkoholische Phase mit einem organischen Lösungsmittel extrahiert, ausgewählt unter den aromatischen Kohlenwasserstoffen.

15. Verfahren nach Anspruch 11, dadurch gekennzeichnet, daß der aromatische Kohlenwasserstoff unter Toluol und Xylol ausgewählt wird.

16. Verfahren nach einem der Ansprüche 2, 3, 4 oder 5, dadurch gekennzeichnet, daß man die durch Dekantieren abgetrennte wäßrig-alkoholische Phase durch Zugabe von Wasser in der Weise verdünnt, daß der Gehalt an Alkohol in der Lösung zwischen 20 und 40 Gew.-% liegt.

17. Verfahren nach einem der Ansprüche 2, 3, 4 oder 5, dadurch gekennzeichnet, daß die unlöslichen Anteile von der verdünnten wäßrig-alkoholischen Lösung durch Filtration, Dekantieren oder Zentrifugieren abgetrennt werden.

18. Verfahren nach einem der Ansprüche 2, 3, 4 oder 5, dadurch gekennzeichnet, daß man das 10-Desacetyl-Baccatin III aus der wäßrig-alkoholischen Phase mit Hilfe eines organischen Lösungsmittels extrahiert.

19. Verfahren nach Anspruch 18, dadurch gekennzeichnet, daß das Lösungsmittel unter den Ethern und den aliphatischen Estern ausgewählt wird.

20. Verfahren nach Anspruch 19, dadurch gekennzeichnet, daß die Ether unter Methyl-tert.-butylether, Ethyl-tert.-butylether, Methyl-n-butylether, Methyl-n-amylether, Ethyl-tert.-amylether, tert.-Butyl-isopropylether, Ethyl-isobutylether, tert.-Butyl-n-propylether und Ethyl-n-hexylether ausgewählt werden.

21. Verfahren nach Anspruch 20, dadurch gekennzeichnet, daß die aliphatischen Ester unter Ethylacetat, Propylacetat, Isopropylacetat, n-Butylacetat, tert.-Butylacetat, Methyl-tert.-butylacetat, tert.-Butylpropionat und tert.-Amylacetat ausgewählt werden.

22. Verfahren nach Anspruch 20, dadurch gekennzeichnet, daß das organische Lösungsmittel Ethylacetat oder n-Butylacetat ist.

23. Verfahren nach einem der Ansprüche 2, 3, 4 oder 5, dadurch gekennzeichnet, daß der das 10-Desacetyl-Baccatin III enthaltende organische Extrakt vollständig oder teilweise durch Destillation, gegebenenfalls unter reduziertem Druck, konzentriert wird.

24. Verfahren nach einem der Ansprüche 1, 2, 3, 4 oder 5, dadurch gekennzeichnet, daß man das 10-Desacetyl-Baccatin III in einem organischen Lösungsmittel selektiv kristallisiert, ausgewählt unter den aliphatischen Nitrilen, gegebenenfalls in Mischung mit einem aliphatischen Alkohol oder einem aliphatischen Ester oder einem aliphatischen Keton.

25. Verfahren nach Anspruch 24, dadurch gekennzeichnet, daß die aliphatischen Nitrile unter Acetonitril und Propionitril ausgewählt werden.

26. Verfahren nach Anspruch 24, dadurch gekennzeichnet, daß der aliphatische Alkohol unter Methanol, Ethanol, Propanol, Isopropanol und n-Butanol ausgewählt wird.

27. Verfahren nach Anspruch 24, dadurch gekennzeichnet, daß der aliphatische Ester unter Ethylacetat, Isopropylacetat, n-Butylacetat und tert.-Butylacetat ausgewählt wird.

28. Verfahren nach Anspruch 24, dadurch gekennzeichnet, daß das Keton unter Aceton, Methylethylketon, Methylpropylketon, Methyl-n-butylketon und Methylisobutylketon ausgewählt wird.

29. Verfahren nach Anspruch 24, dadurch gekennzeichnet, daß die selektive Kristallisation in Acetonitril, gegebenenfalls assoziiert mit Ethanol und/oder Ethylacetat oder n-Butylacetat und/oder Aceton durchgeführt wird.

30. Verfahren nach einem der Ansprüche 1, 2, 3, 4 oder 5, dadurch gekennzeichnet, daß das gereinigte 10-Desacetyl-Baccatin III durch Filtration, Dekantieren oder Zentrifugieren isoliert wird.

31. Verfahren nach einem der Ansprüche 1 bis 30, dadurch gekennzeichnet, daß man das 10-Desacetyl-Baccatin III aus den Blättern der Eibe extrahiert.

32. Verfahren nach Anspruch 31, dadurch gekennzeichnet, daß die Eibe von der Art Taxus baccata, Taxus brevifloria, Taxus canadensis, Taxus cuspidata, Taxus floridana, Taxus media oder Taxus wallichiana stammt.

## Claims

1. Process for obtaining 10-deacetylbaccatin III from various parts of the yew (Taxus sp.), characterized in that:
1) the ground parts of the yew are treated with an aliphatic alcohol so as to obtain an alcoholic extract containing 10-deacetylbaccatin III,
2) the alcoholic extract, optionally concentrated, is diluted with water,
3) the insolubles present in the hydroalcoholic solution obtained are separated off by filtration, sedimentation or centrifuging,
4) virtually all of the alcohol is removed from the hydroalcoholic solution obtained,
5) the 10-deacetylbaccatin III present in the aqueous phase thus obtained is extracted with a suitable organic solvent,
6) the solvent is removed from the organic extract thus obtained,
7) 10-deacetylbaccatin III is selectively crystallized from the residue thus obtained, then
8) the purified 10-deacetylbaccatin III is isolated.

2. Process for obtaining 10-deacetylbaccatin III, characterized in that:
1) the ground parts of the yew are treated with an aliphatic alcohol so as to obtain an alcoholic extract containing 10-deacetylbaccatin III,
2) the alcoholic extract is extracted with an organic solvent,
3) the hydroalcoholic phase obtained is separated off by sedimentation,
4) the hydroalcoholic phase is diluted with water,
5) the insolubles present in the hydroalcoholic solution obtained are separated off by filtration sedimentation or centrifuging,
6) 10-deacetylbaccatin III is extracted from the filtered hydroorganic phase with an organic solvent,
7) the solvent is completely or partly removed from the organic extract containing 10-deacetylbaccatin III,
8) 10-deacetylbaccatin III is selectively crystallized from the solution or the residue obtained in an organic solvent, and
9) 10-deacetylbaccatin III is isolated.

3. Process according to either of claims 1 and 2, characterized in that the alcoholic extract is obtained by stirring the ground and optionally dried parts of the yew in an aliphatic alcohol chosen from methanol, ethanol, propanol, isopropanol and t-butanol.

4. Process according to claim 3, characterized in that the aliphatic alcohol is methanol.

5. Process according to either of claims 1 and 2, characterized in that the ground and optionally dried parts of the yew are obtained by grinding and optionally drying operations which optionally precede or follow the operations of freezing and thawing of the fresh parts of the plant or are interposed between operations of freezing and thawing of the fresh parts of the plant.

6. Process according to one of claims 1, 3, 4 or 5, characterized in that the methanolic extract, optionally concentrated, is diluted with water so that the ratio of the weight of the diluent mixture to the weight of the dry matter present in the alcoholic extract is between 4 and 8 and that the water-alcohol diluent mixture contains 10 to 30 % of alcohol to obtain a hydroalcoholic solution.

7. Process according to one of claims 1, 3, 4 or 5, characterized in that the insolubles are separated from the hydroalcoholic solution by filtration, sedimentation or centrifuging.

8. Process according to one of claims 1, 3, 4 or 5, characterized in that the alcohol is removed from the hydroalcoholic solution by distillation, optionally at reduced pressure.

9. Process according to one of claims 1, 3, 4 or 5, characterized in that 10-deacetylbaccatin III is extracted from the aqueous phase obtained after removal of the alcohol, with an organic solvent.

10. Process according to claim 9, characterized in that the organic solvent is chosen from ethers and aliphatic esters.

11. Process according to claim 10, characterized in that the ethers are chosen from methyl t-butyl ether, ethyl t-butyl ether, methyl n-butyl ether, methyl n-amyl ether, ethyl t-amyl ether, t-butyl isopropyl ether, ethyl isobutyl ether, t-butyl n-propyl ether and ethyl n-hexyl ether.

12. Process according to claim 11, characterized in that the aliphatic esters are chosen from ethyl acetate, propyl acetate, isopropyl acetate, n-butyl acetate, t-butyl acetate, methyl t-butylacetate, t-butyl propionate and t-amyl acetate.

13. Process according to claim 9, characterized in that the organic solvent is ethyl acetate or n-butyl acetate.

14. Process according to one of claims 2, 3, 4 or 5, characterized in that the alcoholic phase is extracted with an organic solvent chosen from aromatic hydrocarbons.

15. Process according to claim 11, characterized in that the aromatic hydrocarbon is chosen from toluene and xylene.

16. Process according to one of claims 2, 3, 4 or 5, characterized in that the hydroalcoholic phase separated off by sedimentation is diluted by addition of water so that the alcohol content in the solution is between 20 and 40 % by weight.

17. Process according to one of claims 2, 3, 4 or 5, characterized in that the insolubles are separated from the dilute hydroalcoholic solution by filtration, sedimentation or centrifuging.

18. Process according to one of claims 2, 3, 4 or 5, characterized in that 10-deacetylbaccatin III is extracted from the hydroalcoholic phase with an organic solvent.

19. Process according to claim 18, characterized in that the solvent is chosen from ethers and aliphatic esters.

20. Process according to claim 19, characterized in that the ethers are chosen from methyl t-butyl ether, ethyl t-butyl ether, methyl n-butyl ether, methyl n-amyl ether, ethyl t-amyl ether, t-butyl isopropyl ether, ethyl isobutyl ether, t-butyl n-propyl ether and ethyl n-hexyl ether.

21. Process according to claim 20, characterized in that the aliphatic esters are chosen from ethyl acetate, propyl acetate, isopropyl acetate, n-butyl acetate, t-butyl acetate, methyl t-butylacetate, t-butyl propionate and t-amyl acetate.

22. Process according to claim 20, characterized in that the organic solvent is ethyl acetate or n-butyl acetate.

23. Process according to one of claims 2, 3, 4 or 5, characterized in that the organic extract containing 10-deacetylbaccatin III is completely or partly concentrated by distillation, optionally at reduced pressure.

24. Process according to one of claims 1, 2, 3, 4 or 5, characterized in that 10-deacetylbaccatin III is selectively crystallized from an organic solvent chosen from aliphatic nitriles optionally mixed with an aliphatic alcohol or an aliphatic ester or an aliphatic ketone.

25. Process according to claim 24, characterized in that the aliphatic nitriles are chosen from acetonitrile and propionitrile.

26. Process according to claim 24, characterized in that the aliphatic alcohol is chosen from methanol, ethanol, propanol, isopropanol and n-butanol.

27. Process according to claim 24, characterized in that the aliphatic ester is chosen from ethyl acetate, isopropyl acetate, n-butyl acetate and t-butyl acetate.

28. Process according to claim 24, characterized in that the ketone is chosen from acetone, methyl ethyl ketone, methyl propyl ketone, methyl n-butyl ketone and methyl isobutyl ketone.

29. Process according to claim 24, characterized in that the selective crystallization is performed in acetonitrile optionally combined with ethanol and/or ethyl or n-butyl acetate and/or acetone.

30. Process according to one of claims 1, 2, 3, 4 or 5, characterized in that the purified 10-deacetylbaccatin III is isolated by filtration, sedimentation or centrifuging.

31. Process according to one of claims 1 to 30, characterized in that 10-deacetylbaccatin III is extracted from yew foliage.

32. Process according to claim 31, characterized in that the yew belongs to the Taxus baccate, Taxus brevifloria, Taxus canadensis, Taxus cuspidata, Taxus floridana, Taxus media or Taxus wallichiana variety.
